Europäisches Patentamt

European Patent Office

Office européen des brevets

(11) Publication number: **0 000 28**

**A1**

(12) **EUROPEAN PATENT APPLICATION**

(21) Application number: 78300093.8

(22) Date of filing: 27.06.78

(51) Int. Cl.²: **A 61 N 1/36,** H 01 R 7/06

(30) Priority: 01.07.77 GB 27625/77
14.06.78 US 915478

(43) Date of publication of application:
**10.01.79 Bulletin 79/1**

(84) Designated Contracting States:
**DE FR GB NL SE**

(71) Applicant: STIMTECH, INC.,
9440 Science Center Drive,
Minneapolis Minnesota (US)

(72) Inventor: Jackson, Frank Tunstall,
22 The Muntings,
Stevenage Herts (GB).

(74) Representative: Colgan, Stephen James et al,
CARPMAELS & RANSFORD 43 Bloomsbury Square,
London W.C.1A, 2RA. (GB)

(54) **Cardiac pacemakers.**

(57) A cardiac pacemaker including implantable housing means (8), electrical signal generating means enclosed within said housing, an electrode conductor (6) for conveying pacing signals to the heart, and apparatus for maintaining sealable electrical interconnection between said generating means and said electrode. Said apparatus comprises a pair of terminal means (2,4) which cooperate through engageable camming surfaces (16,32) to clamp the lead between lead clamping jaws on a collet (20). The pacemaker housing may be bifurcated, with one portion containing the interconnection apparatus, and the other containing the pacemaker electronics and batteries. The two portions are assembled separately and subsequently combined structurally and electrically. One of the terminal means (4) is provided with appropriate slots (40) to engage protuberances from a spanner, thereby facilitating quick and accurate insertion or removal of the electrode conductor.

Fig 1

–1–

## "Cardiac Pacemakers"

This invention relates to cardiac pacemakers and is particularly concerned with the means for making electrical connection between electrical signal generating means and an electrode conductor for conveying pacing signals to the heart.

Implantable cardiac pacemakers include an electronic pulse generator which is implanted in the body and an electrode lead or conductor (catheter) extending from the pulse generator for carrying stimulating pulses to an electrode disposed in or on the heart. Care must be taken over the connection between the electrode lead and the pulse generator (normally a terminal block in the latter), in that the connection must be secure in the body and the interface must be impervious to body fluids. In the past, the electrode lead has been clamped in the terminal block of the pulse generator by a grub screw, with sufficient sealing plugs, sealing rings, gaskets and so forth being provided to exclude body fluids. The grub screw-type fitting is not entirely satisfactory, for example the end of the screw tends to burr over and the clamping which is provided is over only a limited area of contact between the grub screw and the electrode lead.

The foregoing prior art feedthroughs and connectors also present difficulties in terms of speed and accuracy of the catheter installation into or removal from the pacer.

Thus, quite aside from the integrity of the seals, the prior art systems sometime present difficulty to doctors during the process of surgery either to install or to change pacers. Clearly, during the process of installing or replacing pacer, it is important to maintain the continuity of the pacing pulses, while insuring an accurately installed catheter which is maintained in a suitable degree of tension in the feedthrough connection.

The object of the invention is to provide an improved connector system for such apparatus which not only provides a greater area of clamping "contact" of the electrode lead relative to the terminal block, but also, at the same time, enables electrode leads of differing diameters to be accommodated within the same terminal block. With these advantages, sealing of the connection from body fluids can still be maintained.

According to the invention there is provided a cardiac pacemaker including implantable housing means, electrical signal generating means enclosed within said housing, an electrode conductor for conveying pacing signals to the heart, and apparatus for maintaining sealable electrical interconnection between said generating means and said electrode, characterised in that said apparatus comprises:

(a) first terminal means having one end sealed and embedded within said housing, and its other end defining a channel opening to the outside of said housing, said first terminal means having screw threading along at least a portion of said channel, a void at the embedded end of said terminal, and a frustoconical, electrically conductive surface intermediate said void and said threading, said surface tapering outwardly to said threading and being adapted for electrical connection with said generating means; and

second terminal means, disjoint from said housing and adapted for mating connection into said first terminal means, said second terminal means defining an axial channel therethrough for carrying said conductor into a first end, through said axial channel, and out of the opposite end, said second terminal means having a frustocinical camming portion at said opposite end for engagement with the frustoconical surface of said first terminal means, said second terminal means further having a threaded portion intermediate said camming portion and said first end, for engagement with said threading of said first terminal means;

(c) said second terminal means being adapted to carry said conductor into said void, and with increasing engagement of said threaded portion and said threading, to cause said camming portion to grasp said conductor and to establish an electrical circuit between said conductor and said generating means via said frustoconical surface and said camming portion.

Since the pacemaker and electrode conductors can be supplied for implantation separately and since the pacemaker can, within reason, make electrical connection with electrode conductors of different diameters, the pacemaker itself (including the interconnection apparatus) forms a separate aspect of the invention.

In accordance with another feature of the invention, the second terminal means is provided with two or more notches surrounding the entry point of the electrode conductor (the catheter), and a separate spanner has protuberances matable with the notches. The spanner includes a recessed channel to accommodate the catheter, and thereby may be utilized to tighten or loosen the second terminal means and catheter from the first terminal means.

Utilization of connector systems in accordance with the principles of the present invention facilitates a pacer feedthrough scheme whereby the entire pacer housing constitutes two portions, one carrying the interconnection apparatus, and the other carrying the balance of the pacer apparatus. These separate portions are separately manufactured and stored, subsequently to be structurally and electrically interconnected.

Figure 1 illustrates the component parts of a preferred embodiment of the invention, disassembled;

Figure 2 illustrates the components shown in Figure 1 when assembled together to form the connected system; and

Figure 3 shows a conventionally configured pacer having a catheter inserted in accordance with the principles of the present invention;

Figure 4 shows an exploded schematic of the system in accordance with the principles of the present invention, further including a spanner adapted for utilization therewith;

Figure 5 shows a partial cutaway of a pacer installation incorporating the principles of the present invention;

Figure 6 shows a sectional view of a portion of the embodiment of Figure 5, and Figures 9 and 10 show further sections of the embodiment of Figure 6;

Figure 7 shows a view in partial cutaway of an installation employing the principles of the present invention; and

Figure 8 shows a partial section top view of the embodiment of Figure 7.

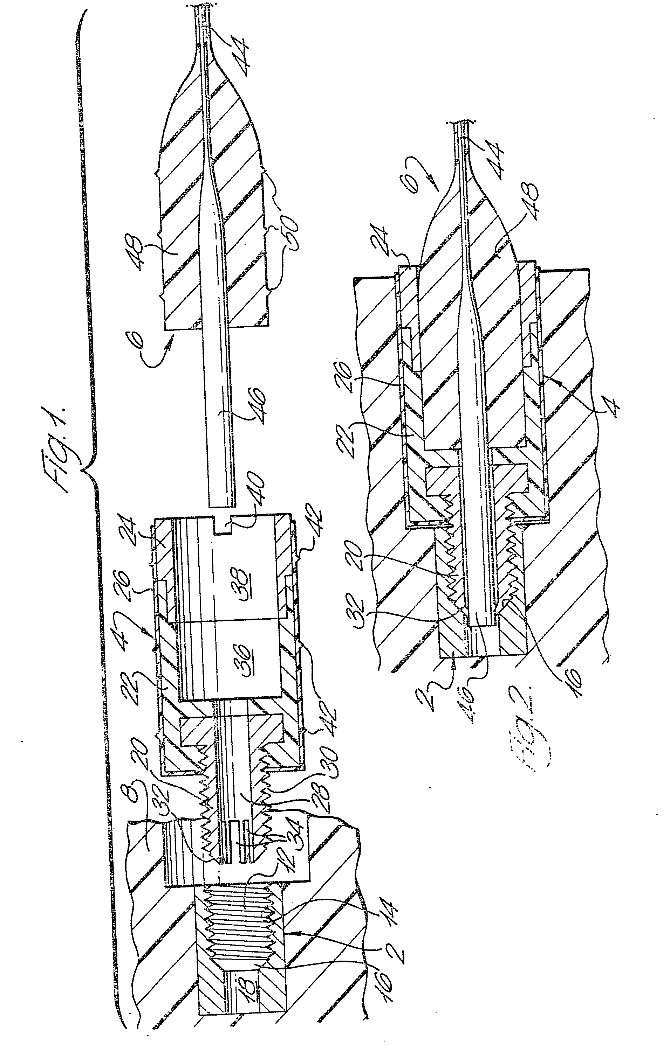

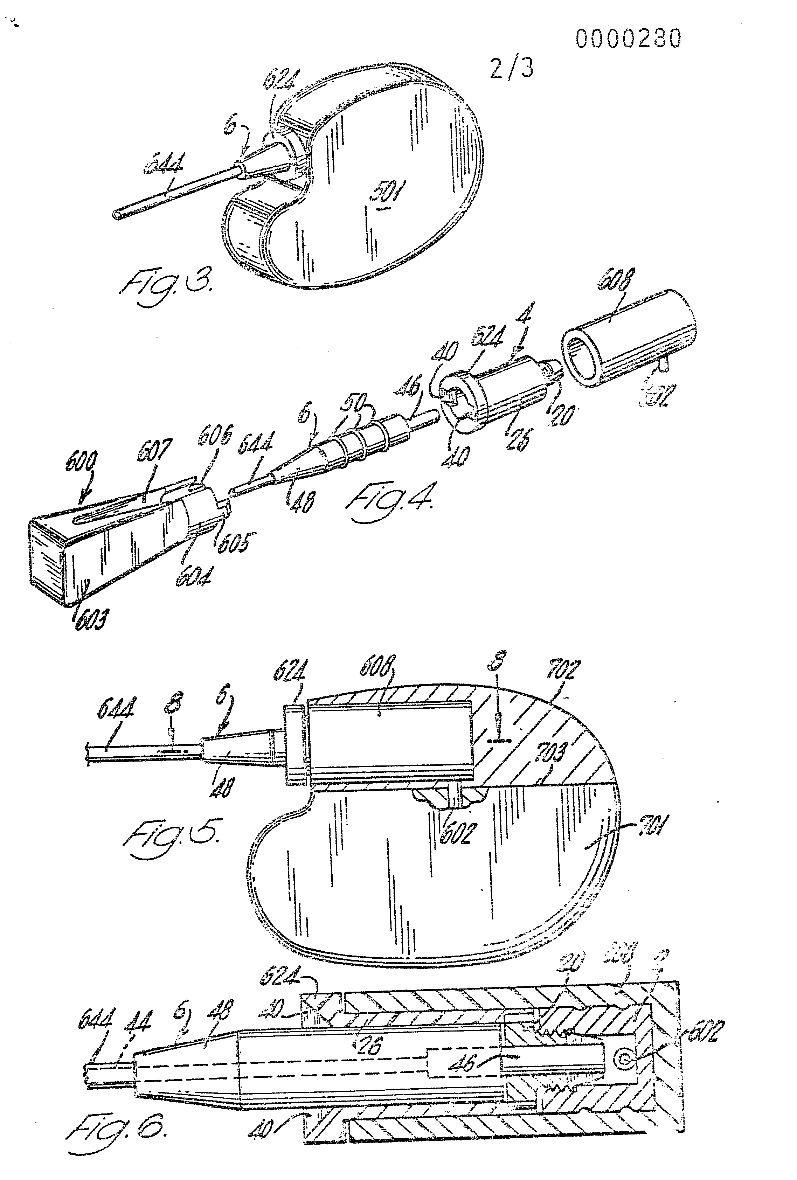

Referring to Figures 1 and 2, the connector system comprises a terminal block 2, a connector 4, and a pacemaker electrode lead 6.

The terminal block 2 is installed within the plastics wall 8 of an implantable pacemaker casing. The terminal block 2 is located at the end of a cylindrical bore 10 leading to the outer surface of the casing; the block is formed of an electrically-conductive metal and is electrically connected to the pacemaker electrical circuitry (not shown). The terminal block has a bore 12 having a threaded portion 14, a tapering, conical portion 16, and a narrower portion 18.

The connector 4 is formed of four components: a metal collet 20; a cylindrical plastics body 22, a metal end portion 24, and an outer plastics sealing sleeve 26. The collet 20 has an axial bore 28, a threaded outer surface 30 terminating in a tapering, conical portion 32. A number of radially-disposed slots 34 extend inwardly from the end of

the collet adjacent the tapering, conical portion 32 to provide clamping jaws for the electrode lead. The opposite end of the collet is embedded within the plastics body 22.

The plastics body 22 has an axial bore 36 concentric with that of the collet and is firmly connected to the metal end portion 24. The latter also is provided with a bore, 38 concentric with that of the collet and plastics body. A pair of radially-opposed slots 40 are provided at the end of the metal end portion 24 remote from the collet.

The outer sealing sleeve 26 surrounds the outer cylindrical surface of the plastics body 22 and metal end portion 24. The diameters of the latter two components are arranged such that the sleeve 26 fits snugly within bore 10 of the pacemaker casing. To ensure a liquid-tight seal, the sleeve 26 is provided with a series of raised circumferential lands 42 which are under compression when the connector is inserted within bore 10 (Figure 2).

The pacemaker electrode lead 6 comprises an electrical conductor 44 which normally leads to the heart and which includes an exposed connector pin portion 46. The conductor 44 is surrounded by a plastics insulating body 48 having a diameter sufficient to fit snugly with the bores 36 and 38 of the plastics body 22 and of the metal end portion 24, respectively. The insulating body is provided with a series of raised circumferential lands 50 which are under compression when the insulating body 48 is inserted within bores 36 and 38, thus providing a liquid-tight seal (Figure 2).

The components of the connector system are assembled as shown in Figure 2, with the electrode lead 6 within the connector 4. The connector pin portion 46 of the former extends to, and slightly beyond the slotted end of the collet 20. The connector is inserted within the bore 10 of the pacemaker casing with the threads of the collet engaging those of the threaded portion 14 of the terminal block 2. To facilitate the engagement of the two threaded portions, a small locking spanner (not shown) having a pair

-6-

of projections may be employed, with the projections engaging the slots 40 on the metal end portion 24. Alternatively, portions may be left protruding from the pacemaker casing for engagement by, e.g. a spanner.

As the threads are engaged, the tapering conical surface 32 of the collet approaches and then contacts the complementary tapering conical portion 16 of the terminal block 2. As the threads are then further engaged, the tapering conical surface 32 is urged inwardly by a camming action relative to tapering conical portion 16 - this movement being enabled by the presence of the slots 34 on the collet. Eventually the inward movement is sufficient for the end of the metal collet adjacent the tapering surfaces to engage and hold firmly the exposed connector pin portion 46 of the electrode lead 6. There then exists a good electrical connection between the terminal block 2 and the electrode lead 6. There also exists, as explained above, a liquid-tight seal between the pacemaker casing and the connector 4 (at the interface of bore 10 and outer sealing sleeve 26) and between the connector 4 and the electrode lead 6 (at the interface of bores 36 and 38 and plastics body 48). Liquids such as body fluids cannot therefore encroach into the pacemaker casing through the bore 10 in the latter and the opening provided by bore 12 in the terminal block 2.

It will be appreciated with the above embodiment that the collet can be employed to clamp electrode leads having connector pin portions of differing diameters. If necessary, collets having different sized axial bores 28 can be supplied so that a choice can be made to select the most appropriate combination for the electrode lead to be employed.

In the above illustrated embodiment the wall of the pacemaker casing has been described as of plastics material. But it could be of another material such as metal when case provision would probably be made for insulating the connector from the electrically-conductive wall).

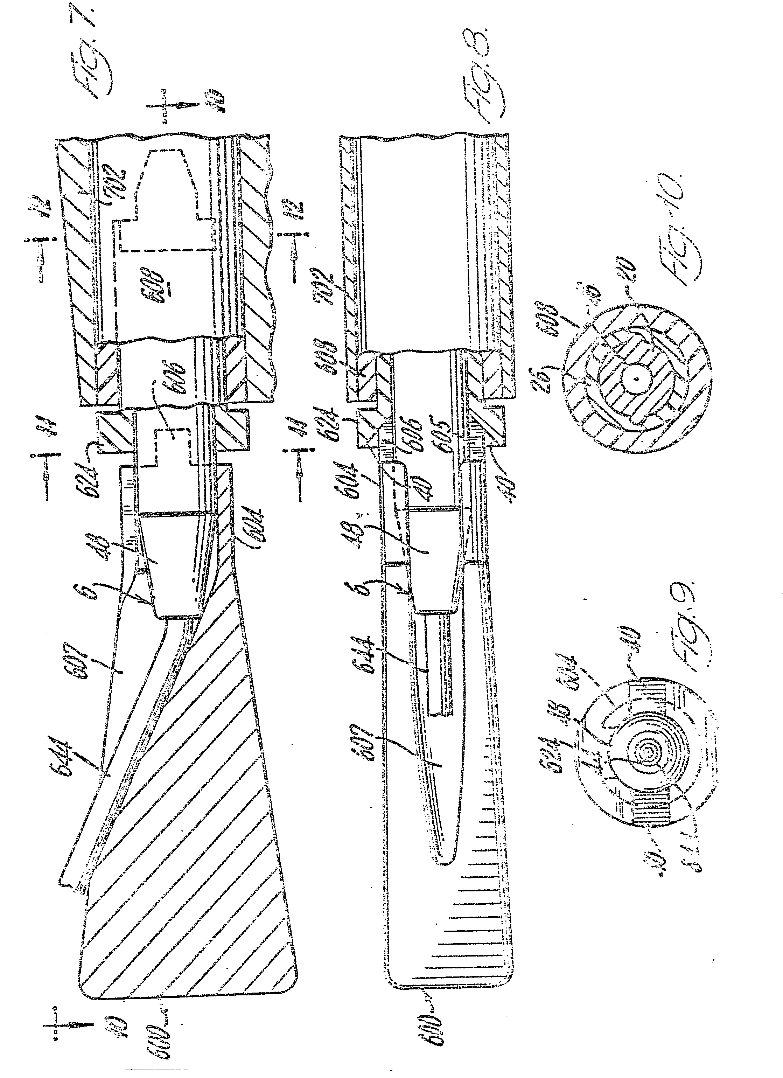

Figure 3 shows an isometric view of a pacer wherein an electrical lead is connected and sealed in place in accordance with the principles of the present invention. The pacer of Figure 3 is encased in a housing 501 of contemporary configuration and design, advantageously composed of a metal or alloy which is non-reactive with body tissues and fluids. The plastic sealing sleeve 26 in accordance with the principles of the present invention defines an outermost circumferential flange 624 which engages the housing 501 when the lead 6 is in place and electrical contact is being made generally as shown in Figure 2. Hence, the catheter 644 extends outwardly, as is known in the art, and into the patient's body such as to the heart for pacing.

With reference to Figure 4, there is shown an exploded view of the elements of Figure 3 incorporating the principles of the present invention. It is understood that the plastics wall 8 of the embodiment of Figure 1 and 2 is shown in Figure 4 as a cylindrical part 608 having an axial bore 708 therein to accommodate the connector 4, and the plastics sleeve 26 thereof, which in turn carries a collet 20 as shown herein. The cylindrical body 608 is fixedly mounted within the housing 501, as shown hereinafter, and includes an outwardly penetrating electrical connection terminal 602, which in turn makes electrical connection through a suitable terminal block (not shown in Figures 3 or 4, but clearly shown in Figure 6), whereby the collet 20 makes electrical contact therewith. The catheter 644 has an insulated body 48 penetrated by pin 46, and the raised circumferential lands 50 insure a seal within the connector 4. A spanner 600 is utilized as described herein to complete installation of the electrode lead 6 within connector 4 and in turn within the cylindrical part 608 and housing 501. To this end, the spanner 600 incorporates a handle portion 603, such as of plastic or metal, an integral rigid end portion 604, and integral protuberances 605 and 606 for engagement with the radially opposed slots 40 of the connector 4. The spanner 600 incorporates a central slot 607 adapted to

-8-

... segment of the insulating body
... electrode lead 6.

Referring next to Figure 5, there is shown an advantageous application of the principles of the present invention, specifically wherein utilization of a collet feedthrough connector in accordance with the present invention facilitates bifurcation of the pacer housing 501 into two separate and distinct parts 701 and 702, generally along the line 703 as shown. The cylindrical plastic receptacle 608 is rigidly and fixedly mounted and encased within portion 702 of the pacer housing, and the electronics and battery aspects (not shown) are encased within the other portion 701. The electrical connector 602, as aforesaid, extends outwardly from cylindrical portion 608 from portion 702, and into the other portion 701 for electrical connection with the circuitry and the like encased therein.

In practice, the top portion 702, including component 608, 602, and the like, may be manufactured and stored separately, to be utilized in conjunction with any circuitry, as desired, which is separately manufactured in portion 701. At the stage of final assembly, portions 701 and 702 are assembled as shown, with electrical contact being established between respective portions 701 and 702 at 602, and the unit is secured and sealed, such as by weldment along line 703. A complete integral unit, such as shown in Figure 3, thereby results.

Figure 6 shows a suitable cross-section of the Figure 5 apparatus, whereby the feedthrough electrical connection may be readily understood. Generally, the Figure 6 view is similar to the previously described view of Figure 2. In Figure 6, the lead 6 penetrates the connector 4, which in turn is fully inserted into the cylindrical portion 608, with the collet 20 engaging terminal block 2 within cylindrical sleeve 608. Electrical conductor 44 from ... 614 penetrates the electrical lead 6, through ... body 48, and within the collet 20. Electrical

continuity thereby is established from conductor 44, to pin 46, collet 20, terminal block 2, and downwardly extending electrical connector pin 602. Cross-sectional views 11-11 and 12-12 are respectively depicted in Figures 9 and 10 thereby affording further clarification of the apparatus shown in Figure 6 and in turn, Figure 5.

Utilization of the spanner 600 may perhaps be better understood upon consideration of Figure 7, and Figure 8 which is a top sectional view thereof takenalong section 10-10. As shown in Figures 7 and 8, the spanner 600 has slots 605 and 607 in matable engagement with radially opposing slots 40 in flange 624 of the connector 4. As shown in Figures 7 and 8, the connector is in the process of insertion, for purposes of forming a connection such as shown in Figures 2 and 6. In such a process, the lead 6, and particularly the tapering portion of insulating body 48 rests within the slot 607 of spanner 600, with catheter lead 644 extending backwardly along the slot and outwardly therefrom. It will be appreciated from consideration of Figures 4, 7 and 8 that the spanner facilitates proper screw tightening of the connector 4--collet 20 combination within the unit as shown.

−1−

Claims:

1. A cardiac pacemaker including implantable housing means (8,501) electrical signal generating means enclosed within said housing, an electrode conductor (6,644) for conveying pacing signals to the heart, and apparatus for maintaining sealable electrical interconnection between said generating means and said electrode characterised in that said apparatus comprises:

(a) first terminal means (2) having one end (18) sealed and embedded within said housing, and its other end defining a channel opening to the outside of said housing, said first terminal means having screw threading (14) along at least a portion of said channel, a void (18) at the embedded end of said terminal, and a frustoconical, electrically conductive surface (16) intermediate said void (18) and said threading (14), said surface tapering outwardly to said threading and being adapted for electrical connection (602) with said generating means; and

(b) second terminal means (4), disjoint from said housing and adapted for mating connection into said first terminal means (2), said second terminal means defining an axial channel (28) therethrough for carrying said conductor (6,644) into a first end, through said axial channel, and out of the opposite end, said second terminal means having a frustoconical camming portion (32) at said opposite end for engagement with the frustconical surface of said first terminal means, said second terminal means further having a threaded portion (30) intermediate said camming portion and said first end, for engagement with said threading of said first terminal means;

(c) said second terminal means (4) being adapted to carry said conductor (6,644) into said void (18), and with increasing engagement of said threaded portion (30) and said threading (4), to cause said camming portion(32) to grasp said conductor (6,644) and to establish an electrical circuit between said conductor and said generating means via said frustoconical surface (16) and said camming portion (32).

2. A cardiac pacemaker as claimed in claim 1 characterised in that said second terminal means (4) includes a radially extending flange (624) about said one end, said flange defining a plurality of radially disposed slots (40) therein; said pacemaker being assocated with spanner means (600) for tightening said second terminal means (4) into said first terminal means (2), said spanner means (600) having plural protuberances (605,606) in spatial correspondence with said radially disposed slots (40), said spanner means (600) including a tapering trough (607) for accommodating said electrode conductor (6,644) when said protuberances (605,606) and said slots (40) engage one another.

3. A cardiac pacemaker as claimed in claim 1 or 2 characterised in that said first terminal means (2) comprises a first discrete portion (702) of said housing, separate from but matable with a second discrete portion (701) of said housing for enclosing said generating means, said discrete portions being sealably attachable to one another (703), said second terminal means (2) including a connector (602) for electrical interconnection between said discrete portions.

4. A cardiac pacemaker as claimed in any of claims 1 to 3 characterised in that said second terminal means (4) includes a portion, intermediate said threaded portion and said one end, defining raised lands (42) for compressive sealing engagement with inner surfaces of said channel of said first terminal means.

5. A cardiac pacemaker as claimed in any of claims 1 to 4 characterised in that said second terminal means (4) is adapted to receive sealably an electrode connector having an insulating body (48) surrounding a conductor pin (46), said axial channel including successive segments (28,36) of increasing diameter to accommodate respectively said pin (46) freely, and said insulating body compressively sealed.

Fig.1.

Fig.2.

Fig. 3.

Fig. 4.

Fig. 5.

Fig. 6.

FIG. 7.

FIG. 8.

FIG. 9.

FIG. 10.

0000280

0000280

# European Patent Office

## EUROPEAN SEARCH REPORT

Application number

EP 78 30 0093

| | DOCUMENTS CONSIDERED TO BE RELEVANT | | CLASSIFICATION OF THE APPLICATION (Int. Cl.²) |
|---|---|---|---|
| Category | Citation of document with indication, where appropriate, of relevant passages | Relevant to claim | |
| | CH - A - 457 639 (CONTRAVES) <br> * Column 1, lines 1-11; column 2, lines 57-67; column 4, lines 1-11 * | 1 | A 61 N 1/36 <br> H 01 R 7/06 |
| | FR - A - 2 062 750 (CAMERA) <br> * Page 2, lines 23-41; page 3, lines 1-3 * | 1 | |
| | FR - A - 2 224 894 (LINDSAY) <br> * Page 3, lines 27-30, 33-36; page 4, lines 18-26, 33-38; page 5, lines 18-28 * | 1 | **TECHNICAL FIELDS SEARCHED (Int.Cl.²)** <br><br> A 61 N 1/36 <br> H 01 R 17/12 <br> H 01 R 5/00 <br> H 01 R 7/00 <br> H 01 R 43/00 <br> H 01 R 7/06 |
| | GB - A - 1 304 140 (ITT) <br> * Page 3, lines 33-73; figure 3 * | 1 | |
| | US - A - 3 760 332 (BERKOVITS) <br> * Column 1, lines 3-18; column 2, lines 23-30; column 4, lines 45-47; figure 4 * | 1,4,5 | |
| | US - A - 4 010 757 (JULA) <br> * Column 2, lines 55-58; column 3, lines 28-34; figure 3 * | 2 | **CATEGORY OF CITED DOCUMENTS** <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| | US - A - 2 973 219 (ZALK) <br> * Column 2, lines 49-71 * <br> ./. | 2 | |

&: member of the same patent family, corresponding document

The present search report has been drawn up for all claims

| Place of search | Date of completion of the search | Examiner |
|---|---|---|
| The Hague | 10-10-1978 | TIELEMANS |

EPO Form 1503.1 03.79

0000280

| | DOCUMENTS CONSIDERED TO BE RELEVANT | | CLASSIFICATION OF THE APPLICATION (Int. Cl.²) |
|---|---|---|---|
| Category | Citation of document with indication, where appropriate, of relevant passages | Relevant to claim | |
| | US − A − 3 842 842 (KENNY) <br> * Column 1, lines 22-40, 54-65 * | 3 | |
| | | | TECHNICAL FIELDS SEARCHED (Int. Cl.²) |

Form 1503.1